# EUROPEAN PATENT SPECIFICATION

(11) **EP 3 710 058 B1**
(45) Date of publication and mention of the grant of the patent: **09.10.2024**
(21) Application number: 18808075.8
(22) Date of filing: 15.11.2018
(51) Int. Cl.: A61K 31/05, A61K 31/352, A61K 45/06, A61P 25/08, A61K 36/185

(54) **USE OF CANNABIDIOL IN ADDITION TO LAMOTRIGINE FOR THE TREATMENT OF SEIZURES ASSOCIATED WITH LENNOX-GASTAUT SYNDROME**
VERWENDUNG VON CANNABIDIOL ZUSÄTZLICH ZU LAMOTRIGIN ZUR BEHANDLUNG VON MIT DEM LENNOX-GASTAUT-SYNDROM VERBUNDENEN ANFÄLLEN
UTILISATION DU CANNABIDIOL EN PLUS DE LAMOTRIGINE DANS LE TRAITEMENT DE CRISES ASSOCIÉES AU SYNDROME DE LENNOX-GASTAUT

(30) Priority: 15.11.2017 GB 201718862
(43) Date of publication of application: 23.09.2020
(73) Proprietor: Jazz Pharmaceuticals Research UK Limited, Sittingbourne Kent ME9 8AG (GB)
(72) Inventor: GUY, Geoffrey, Sittingbourne Kent ME9 8AG (GB); KNAPPERTZ, Volker, Sittingbourne Kent ME9 8AG (GB)
(74) Representative: HGF
(86) International application number: PCT/GB2018/053315
(87) International publication number: WO 2019/097238

(56) References cited:
- GB-A- 2 531 282
- US-A1- 2017 224 634
- OSTENDORF ADAM ET AL: "Treatment-resistant Lennox-Gastaut syndrome: therapeutic trends, challenges and future directions", NEUROPSYCHIATRIC DISEASE AND TREATMENT, vol. Volume 13, 1 April 2017 (2017-04-01), pages 1131 - 1140, XP055939166, Retrieved from the Internet <URL:https://www.dovepress.com/getfile.php?fileID=36134> [retrieved on 20220706], DOI: 10.2147/NDT.S115996
- O DEVINSKY ET AL: "Efficacy and safety of Epidiolex (cannabidiol) in children and young adults with treatment-resistant epilepsy: Initial data from expanded access program", 1 January 2015 (2015-01-01), XP055553292, Retrieved from the Internet <URL:https://www.aesnet.org/meetings_events/annual_meeting_abstracts/view/2414222> [retrieved on 20190207]
- DEVINSKY ORRIN ET AL: "Cannabidiol in patients with treatment-resistant epilepsy: an open-label interventional trial", LANCET NEUROLOGY, LANCET PUBLISHING GROUP, LONDON, GB, vol. 15, no. 3, 24 December 2015 (2015-12-24), pages 270 - 278, XP029415431, ISSN: 1474-4422, DOI: 10.1016/S1474-4422(15)00379-8
- ALICIA CICCONE: "Drop Seizure Frequency in Lennox-Gastaut Decreased With Cannabidiol", NEUROLOGY ADVISOR, 26 April 2017 (2017-04-26), pages 1 - 3, XP055544182, Retrieved from the Internet <URL:https://www.neurologyadvisor.com/aan-2017-coverage/aan-2017-cannabidiol-reduces-drop-seizures-in-lennox-gastaut-syndrome/article/652931/> [retrieved on 20190117]
- WIRRELL E ET AL: "Cannabidiol (CBD) significantly reduces drop and total seizure frequency in lennox gastaut syndrome (LGS): Results of a dose ranging, multicenter, randomized, double blind, placebo controlled trial (GWPCARE3)", 1 October 2017 (2017-10-01), XP002788724, Retrieved from the Internet <URL:http://ir.gwpharm.com/static-files/d489e19a-7769-4dcb-97fc-60ed923b0a2d>

## Description

### FIELD OF THE INVENTION

The present invention relates to cannabidiol (CBD) for use in the treatment of drop seizures associated with Lennox-Gastaut syndrome (LGS) in patients who are deemed to be treatment failures on lamotrigine; wherein the CBD is in the form of a highly purified extract of cannabis which comprises at least 98% (w/w) CBD or is present as a synthetic compound .

In particular the use of CBD was found to provide a statistically significant reduction in both drop seizures and total seizure frequency in patients who have tried and failed lamotrigine or those who were currently taking lamotrigine but have uncontrolled seizures.

The AEDs which have been shown to be treatment failures is lamotrigine.

Preferably the CBD used is in the form of a highly purified extract of cannabis such that the CBD is present at greater than 98% of the total extract (w/w) and the other components of the extract are characterised. In particular the cannabinoid tetrahydrocannabinol (THC) has been substantially removed, to a level of not more than 0.15% (w/w) and the propyl analogue of CBD, cannabidivarin, (CBDV) is present in amounts of up to 1%. Alternatively, the CBD may be a synthetically produced CBD.

### BACKGROUND TO THE INVENTION

Epilepsy occurs in approximately 1% of the population worldwide, (Thurman *et al.,* 2011) of which 70% are able to adequately control their symptoms with the available existing anti-epileptic drugs (AEDs). However, 30% of this patient group, (Eadie *et al.,* 2012), are unable to obtain seizure freedom from the AED that are available and as such are termed as suffering from intractable or "treatment-resistant epilepsy" (TRE).

Intractable or treatment-resistant epilepsy was defined in 2009 by the International League Against Epilepsy (ILAE) as *"failure of adequate trials of two tolerated and appropriately chosen and used AED schedules (whether* as *monotherapies or in combination) to achieve sustained seizure freedom"* (Kwan *et al.,* 2009).

Individuals who develop epilepsy during the first few years of life are often difficult to treat and as such are often termed treatment-resistant. Children who undergo frequent seizures in childhood are often left with neurological damage which can cause cognitive, behavioral and motor delays.

Childhood epilepsy is a relatively common neurological disorder in children and young adults with a prevalence of approximately 700 per 100,000. This is twice the number of epileptic adults per population.

When a child or young adult presents with a seizure, investigations are normally undertaken in order to investigate the cause. Childhood epilepsy can be caused by many different syndromes and genetic mutations and as such diagnosis for these children may take some time.

The main symptom of epilepsy is repeated seizures. In order to determine the type of epilepsy or the epileptic syndrome that a patient is suffering from, an investigation into the type of seizures that the patient is experiencing is undertaken. Clinical observations and electroencephalography (EEG) tests are conducted and the type(s) of seizures are classified according to the ILAE classification described below.

The International classification of seizure types proposed by the ILAE was adopted in 1981 and a revised proposal was published by the ILAE in 2010 and has not yet superseded the 1981 classification. The 2010 proposal for revised terminology includes the proposed changes to replace the terminology of partial with focal. In addition, the term "simple partial seizure" has been replaced by the term "focal seizure where awareness / responsiveness is not impaired" and the term "complex partial seizure" has been replaced by the term "focal seizure where awareness / consciousness is impaired".

Generalised seizures, where the seizure arises within and rapidly engages bilaterally distributed networks, can be split into six subtypes: Tonic-Clonic (grand mal) seizures; Absence (petit mal) Seizures; Clonic Seizures; Tonic Seizures; Atonic Seizures and Myoclonic Seizures.

Focal (partial) seizures where the seizure originates within networks limited to only one hemisphere, are also split into sub-categories. Here the seizure is characterized according to one or more features of the seizure, including aura, motor, autonomic and awareness / responsiveness. Where a seizure begins as a localized seizure and rapidly evolves to be distributed within bilateral networks this seizure is known as a Bilateral convulsive seizure, which is the proposed terminology to replace Secondary Generalised Seizures (generalized seizures that have evolved from focal seizures and are no longer remain localized).

Epileptic syndromes often present with many different types of seizure and identifying the types of seizure that a patient is suffering from is important as many of the standard AEDs are targeted to treat or are only effective against a given seizure type / sub-type.

One such childhood epilepsy syndrome is Lennox-Gastaut syndrome (LGS). LGS is a severe form of epilepsy, where seizures usually begin before the age of 4. Seizure types, which vary among patients, include tonic (stiffening of the body, upward deviation of the eyes, dilation of the pupils, and altered respiratory patterns), atonic (brief loss of muscle tone and consciousness, causing abrupt falls), atypical absence (staring spells), and myoclonic (sudden muscle jerks). There may be periods of frequent seizures mixed with brief, relatively seizure-free periods.

Seizures in LGS are often described as "drop seizures". Such drop seizures are defined as an attack or spell (atonic, tonic or tonic-clonic) involving the entire body, trunk or head that led or could have led to a fall, injury, slumping in a chair or hitting the patient's head on a surface.

Most patients with LGS experience some degree of impaired intellectual functioning or information processing, along with developmental delays, and behavioural disturbances.

LGS can be caused by brain malformations, perinatal asphyxia, severe head injury, central nervous system infection and inherited degenerative or metabolic conditions. In 30-35% of cases, no cause can be found.

The first line treatment for drop seizures, including the treatment of drop seizures in patients with LGS, usually comprises a broad-spectrum AED, such as sodium valproate often in combination with rufinamide or lamotrigine. Other AEDs that may be considered include felbamate, clobazam and topiramate.

AEDs such as carbamezapine, gabapentin, oxcarbazepine, pregabalin, tiagabineor and vigabatrin are contra-indicated in drop seizures.

Common AEDs defined by their mechanisms of action are described in the following tables:

**Table 1. Examples of narrow spectrum AEDs**

| **Narrow-spectrum AED** | **Mechanism** | **Indication** |
|---|---|---|
| Phenytoin | Sodium channel | Complex partial |
| | | Tonic-clonic |
| Phenobarbital | GABA / Calcium channel | Partial seizures |
| | | Tonic-clonic |
| Carbamazepine | Sodium channel | Partial seizures |
| | | Tonic-clonic |
| | | Mixed seizures |
| Oxcarbazepine | Sodium channel | Partial seizures |
| | | Tonic-clonic |
| | | Mixed seizures |
| Gabapentin | Calcium channel | Partial seizures |
| | | Mixed seizures |
| Pregabalin | Calcium channel | Adjunct therapy for partial seizures with or without secondary generalisation |
| Lacosamide | Sodium channel | Adjunct therapy for partial seizures |
| Vigabatrin | GABA | Secondarily generalized tonic-clonic seizures |
| | | Partial seizures |
| | | Infantile spasms due to West syndrome |

**Table 2. Examples of broad spectrum AEDs**

| **Broad-spectrum AED** | **Mechanism** | **Indication** |
|---|---|---|
| Valproic acid | GABA / Sodium channel | First-line treatment for tonic-clonic seizures, absence seizures and myoclonic seizures |
| | | Second-line treatment for partial seizures and infantile spasms. |
| | | Intravenous use in status epilepticus |
| Lamotrigine | Sodium channel | Partial seizures |
| | | Tonic-clonic |
| | | Seizures associated with |
| | | Lennox-Gastaut syndrome |
| Ethosuximide | Calcium channel | Absence seizures |
| Topiramate | GABA / Sodium channel | Seizures associated with Lennox-Gastaut syndrome |
| Zonisamide | GABA / Calcium /Sodium channel | Adjunctive therapy in adults with partial-onset seizures |
| | | Infantile spasm |
| | | Mixed seizure |
| | | Lennox-Gastaut syndrome |
| | | Myoclonic |
| | | Generalised tonic-clonic seizure |
| Levetiracetam | Calcium channel | Partial seizures |
| | | Adjunctive therapy for partial, myoclonic and tonic-clonic seizures |
| Clonazepam | GABA | Typical and atypical absences |
| | | Infantile myoclonic |
| | | Myoclonic seizures |
| | | Akinetic seizures |
| Rufinamide | Sodium channel | Adjunctive treatment of partial seizures associated with Lennox-Gastaut syndrome |

**Table 3. Examples of AEDs used specifically in childhood epilepsy**

| **AED** | **Mechanism** | **Indication** |
|---|---|---|
| Clobazam | GABA | Adjunctive therapy in complex partial seizures |
| | | Status epilepticus |
| | | Myoclonic |
| | | Myoclonic-absent |
| | | Simple partial |
| | | Complex partial |
| | | Absence seizures |
| | | Lennox-Gastaut syndrome |
| Stiripentol | GABA | Severe myoclonic epilepsy in infancy (Dravet syndrome) |

The present invention describes surprising data from two placebo-controlled studies of CBD as treatment for seizures associated with LGS. The CBD was used as add-on treatment in patients who were defined as treatment-resistant. Patients had previously tried and stopped using (failed) a median of 6 AEDs, and were being maintained on a median of 3 AEDs.

Despite this intensive treatment regimen, the median number of drop seizures at baseline was in excess of 75 per month in both studies. These were patient populations with a high unmet medical need, who had already tried and failed multiple AEDs. In many cases, the AEDs they were being maintained on included those approved for the treatment of LGS; rufinamide, lamotrigine or topiramate.

The data collected on patients who were deemed to be treatment failures to one or more of the existing approved drugs for LGS showed that the use of CBD in combination with these medications resulted in a statistically significant reduction in both drop seizures and total seizure frequency.

In August 2017, Gaston *et al.* described that when CBD was administered to patients with epilepsy in an open label study, it was found that the serum levels of some AEDs increased in the presence of CBD. The study did not find that such an increase resulted in a reduction of seizures in patients that had been deemed to be treatment failures.

GB2531282A is directed to the use of cannabinoids in the treatment of epilepsy.

US2017/224634 A1 is directed to stable cannabinoid formulations.

Devinsky et al., 1 January 2015, (retrieved from the URL: https://www.aesnet.org/meetings_events/annual_meeting_abstracts/view/2414222) is a meeting abstract entitled "Efficacy and safety of Epidiolex (cannabidiol) in children and young adults with treatment-resistant epilepsy: initial data from an expanded access program".

Devinsky et al., Lancet Neurology, Lancet publishing group, London, GB, vol. 15, no. 3, 24 December 2015, pages 270-278, is a journal article describing the use of cannabidiol in patients with treatment-resistant epilepsy: an open-label interventional trial.

Ciccone, A., Neurology Advisor, 26 April 2017, pages 1-3, is an article entitled "Drop Seizure Frequency in Lennox-Gastaut Decreased with Cannabidiol".

Wirrell et al., Embase database accession no. EMB-622416344; and Annals of Neurology 20171001, John Wiley and sons Inc., vol. 82, no. Supplement 21, 1 October 2017, pages S279 20171004 to 20171007 Kansas City, MO-S280 conference, is a poster entitled "Cannabidiol (CBD) significantly reduces drop and total seizure frequency in lennox gastaut syndrome (LGS): Results of a dose ranging, multicenter, randomized, double blind, placebo controlled trial (GWPCARE3)".

Ostendorf et al., Neuropsychiatric disease and treatment, vol. 13, 1 April 2017, pages 1131-1140, is an article entitled "Treatment-resistant Lennox-Gastaut syndrome: therapeutic trends, challenges and future directions".

Nice Nation Institute For Health and Care Excellence: "Epilepsies: diagnosis and management (CG137)", 1 February 2020, pages 1-90, are clinical guidelines for the diagnosis and management of epilepsies.

### BRIEF SUMMARY OF THE DISCLOSURE

In accordance with a first aspect of the present invention there is provided cannabidiol (CBD) for use in the treatment of drop seizures associated with Lennox-Gastaut syndrome (LGS) characterised in that the patient with LGS is deemed to be a treatment failure on lamotrigine; wherein the CBD is in the form of a highly purified extract of cannabis which comprises at least 98% (w/w) CBD or is present as a synthetic compound.

Preferably the CBD is in the form of a highly purified extract of cannabis which comprises at least 98% (w/w) CBD. Alternatively, the CBD is present as a synthetic compound.

Preferably the extract comprises less than 0.15% THC. More preferably the extract further comprises up to 1% CBDV.

Preferably the dose of CBD is below 50 mg/kg/day, more preferably below 30 mg/kg/day, more preferably still the dose of CBD is 20 mg/kg/day or greater, more preferably still the dose of CBD is 10 mg/kg/day or greater.

In accordance with a second aspect of the present invention there is provided CBD for use in a method of treating drop seizures associated with Lennox-Gastaut syndrome (LGS) comprising administering cannabidiol (CBD) to a subject diagnosed with LGS who are deemed to be a treatment failure on lamotrigine; wherein the CBD is in the form of a highly purified extract of cannabis which comprises at least 98% (w/w) CBD or is present as a synthetic compound.

Preferably the subject is a human.

### DEFINITIONS

Definitions of some of the terms used to describe the invention are detailed below:

The cannabinoids described in the present application are listed below along with their standard abbreviations.

**Table 4. Cannabinoids and their abbreviations**

| | | |
|---|---|---|
| CBD | Cannabidiol | 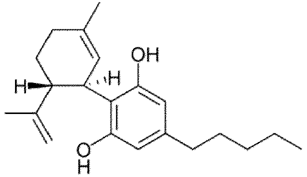 |
| CBDA | Cannabidiolic acid | 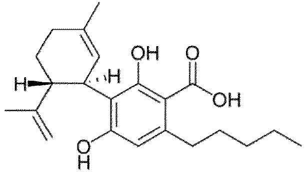 |
| CBDV | Cannabidivarin | 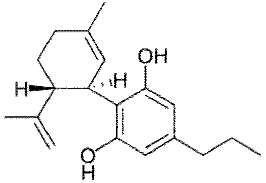 |
| CBDVA | Cannabidivarinic acid | 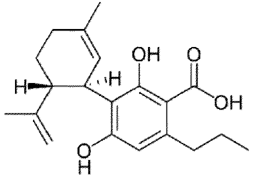 |
| THC | Tetrahydrocannabinol | 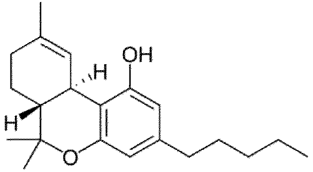 |

The table above is not exhaustive and merely details the cannabinoids which are identified in the present application for reference. So far over 60 different cannabinoids have been identified and these cannabinoids can be split into different groups as follows: Phytocannabinoids; Endocannabinoids and Synthetic cannabinoids (which may be novel cannabinoids or synthetically produced phytocannabinoids or endocannabinoids).

"Phytocannabinoids" are cannabinoids that originate from nature and can be found in the cannabis plant. The phytocannabinoids can be isolated from plants to produce a highly purified extract or can be reproduced synthetically.

"Highly purified cannabinoid extracts" are defined as cannabinoids that have been extracted from the cannabis plant and purified to the extent that other cannabinoids and non-cannabinoid components that are co-extracted with the cannabinoids have been substantially removed, such that the highly purified cannabinoid is greater than or equal to 98% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

"Treatment-resistant epilepsy" (TRE) or "intractable epilepsy" is defined as per the ILAE guidance of 2009 as epilepsy that is not adequately controlled by trials of one or more AED.

"Childhood epilepsy" refers to the many different syndromes and genetic mutations that can occur to cause epilepsy in childhood. Examples of some of these are as follows: Dravet Syndrome; Myoclonic-Absence Epilepsy; Lennox-Gastaut syndrome; Generalized Epilepsy of unknown origin; CDKL5 mutation; Aicardi syndrome; tuberous sclerosis complex; bilateral polymicrogyria; Dup15q; SNAP25; and febrile infection related epilepsy syndrome (FIRES); benign rolandic epilepsy; juvenile myoclonic epilepsy; infantile spasm (West syndrome); and Landau-Kleffner syndrome. The list above is non-exhaustive as many different childhood epilepsies exist.

"Drop seizures" are defined as a seizure involving the entire body, trunk or head that led or could have led to a fall, injury, slumping in a chair or hitting the patient's head on a surface. Seizure types classed as resulting in a drop seizure are atonic, tonic or tonic-clonic seizures.

"Treatment failure" is defined as patients who were currently taking an AED but continued to have uncontrolled seizures, or had previously taken and stopped treatment with an AED due to lack of efficacy in controlling seizures.

### DETAILED DESCRIPTION

### PREPARATION OF HIGHLY PURIFIED CBD EXTRACT

The following describes the production of the highly-purified (>98% w/w) cannabidiol extract which has a known and constant composition was used in the Examples below.

In summary the drug substance used is a liquid carbon dioxide extract of high-CBD containing chemotypes of *Cannabis sativa* L. which had been further purified by a solvent crystallization method to yield CBD. The crystallisation process specifically removes other cannabinoids and plant components to yield greater than 98% CBD. Although the CBD is highly purified because it is produced from a cannabis plant rather than synthetically there is a small number of other cannabinoids which are co-produced and co-extracted with the CBD. Details of these cannabinoids and the quantities in which they are present in the medication are as described in Table 5 below.

**Table 5: Composition of highly purified CBD extract**

| **Cannabinoid** | **Concentration** |
|---|---|
| CBD | > 98% w/w |
| CBDA | NMT 0.15% w/w |
| CBDV | NMT 1.0% w/w |
| Δ⁹ THC | NMT 0.15% w/w |
| CBD-C4 | NMT 0.5% w/w |

| | |
|---|---|
| > - greater than NMT - not more than | |

### EXAMPLE 1: EFFICACY OF CANNABIDIOL IN THE TREATMENT OF LENNOX-GASTAUT SYNDROME (LGS) IN PATIENTS DEEMED TO BE ANTI-EPILEPTIC DRUG (AED) TREATMENT FAILURES

Currently only four products have been authorised in the EU for the treatment of LGS. These medications are rufinamide, lamotrigine, topiramate and felbamate. Details of these can be found in Table 6 below.

**Table 6: Summary of Approved Treatment Options for Lennox-Gastaut Syndrome**

| **Treatment** | **Authorisation in the EU** | **Licensed use in LGS (www.medicines.org.uk or EMA Website)** |
|---|---|---|
| **Rufinamide** | Approved via the centralised procedure on 16/01/2007 | Adjunctive therapy in the treatment of seizures associated with Lennox-Gastaut syndrome in patients 4 years of age and older. |
| **Lamotrigine** | First approval August 1997 | Seizures associated with Lennox-Gastaut syndrome. |
| **Topiramate** | Nationally approved; Date of first authorisation in UK: 18/07/1995 | Adjunctive therapy in children aged 2 years and above, adolescents and adults with partial onset seizures with or without secondary generalisation or primary generalised tonic-clonic seizures and for the treatment of seizures associated with Lennox-Gastaut syndrome. |
| **Felbamate** | Nationally approved: Date of first authorisation in France: 16/05/1994 | Adjunctive therapy in the treatment of partial and generalised seizures associated with Lennox-Gastaut syndrome in children. |
| | | For use only in those patients who respond inadequately to alternative treatments and whose epilepsy is so severe that a substantial risk of aplastic anaemia and/or liver failure is deemed acceptable |

The UK's National Institute for Clinical Excellence (NICE) have produced a recommended pathway for the treatment of LGS (NICE, 2016). NICE have suggested that first line treatment is sodium valproate. If this is not effective or tolerated then lamotrigine should be prescribed as adjunctive treatment. Other AEDs that may be used are rufinamide, topiramate and felbamate. Usually, it takes a combination of more than one AED to gain any seizure control.

All four compounds authorised in the EU specifically for the treatment of LGS all seems to act via sodium channel blockade (see Table 7). Since CBD has a different mode of action to these treatments, it offers prescribers and patients a viable alternative for the treatment of this disease, which is notoriously unresponsive to conventional anti-epileptic treatments.

**Table 7: Modes of action of antiepileptic drugs authorised in the EU specifically to treat Lennox-Gastaut Syndrome**

| **Drug** | **Mode of action** |
|---|---|
| Rufinamide | Uncertain, but thought to modulate the activity of sodium channels, prolonging their inactive state |
| Lamotrigine | Is a use- and voltage-dependent blocker of voltage-gated sodium channels. It inhibits sustained repetitive firing of neurons and inhibits release of glutamate (the neurotransmitter which plays a key role in the generation of epileptic seizures). |
| Topiramate | Full mode of action unknown but thought to contribute to the anti-seizure effects via: |
| | state-dependent sodium channel blocking action |
| | potentiation of the activity of GABA |
| Felbamate | A sodium channel inactivator. |

In two placebo-controlled studies of CBD as a treatment for seizures associated with LGS, cannabidiol was used as add-on treatment in patients who were defined as treatment-resistant. Patients had previously tried and stopped using a median of 6 AEDs, and were being maintained on a median of 3 AEDs.

The first study was a 1:1 randomised, double-blind, 14-week comparison of cannabidiol oral solution (CBD-OS) versus placebo. The treatment period consisted of a two-week titration period followed by a 12-week maintenance period. The treatment period was followed by a 10-day taper period and a four-week follow-up period. The study aimed to determine the efficacy, safety and tolerability of 20 mg/kg/day cannabidiol compared with placebo.

The second study was a 1:1:1 randomised, double-blind, 14-week comparison of two dose levels of cannabidiol (10 mg/kg/day and 20 mg/kg/day) versus placebo. The treatment period consisted of a two-week titration period followed by a 12-week maintenance period. The treatment period was followed by a 10-day taper period and a four-week follow-up period. The study aimed to determine the efficacy, safety and tolerability of two dose levels of CBD-OS compared with placebo. Patients in the placebo group were split into two equivalent cohorts; half receiving 10 mg/kg/day dosing volumes and half receiving 20 mg/kg/day dosing volumes.

In order to ensure that caregivers were able to interpret a seizure correctly, all caregivers underwent pre-study training, and their description and classification of a seizure was independently verified by a committee appointed by the Epilepsy Study Consortium

Despite the number of medications, the patients were already taking, the median number of drop seizures at baseline was in excess of 75 per month in both studies. These were patient populations with a high unmet medical need, who had already tried and failed multiple AEDs. In many cases, the AEDs they were being maintained on included those approved for the treatment of LGS; rufinamide, lamotrigine or topiramate.

An analysis of the change in seizure frequency for CBD vs placebo in patients who were currently taking, or had previously taken and stopped, each of rufinamide, lamotrigine or topiramate; felbamate was undertaken. Such patients can be reasonably defined as AED treatment failures.

### Rufinamide

Tables 8 to 10 below show the results in patients who have tried and failed rufinamide, or those who are currently taking it but have uncontrolled seizures. There was a significantly greater reduction in drop and total seizure frequency and a significantly greater proportion of patients with a ≥ 50% reduction in drop seizure frequency at the 20 mg/kg/day dose of cannabidiol compared with placebo.

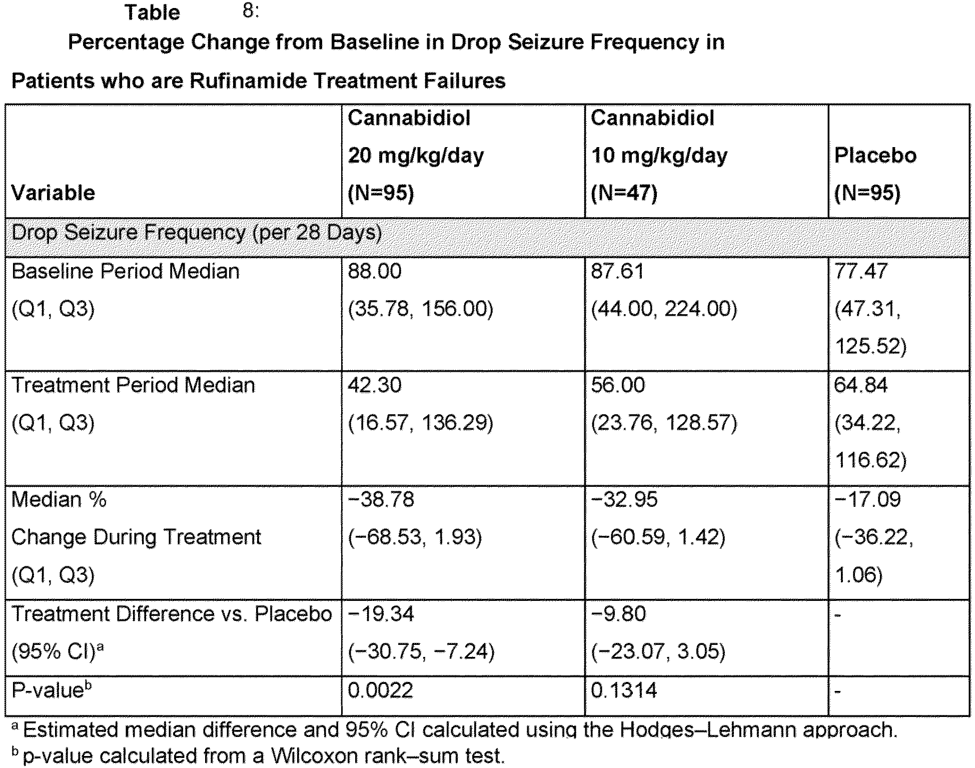

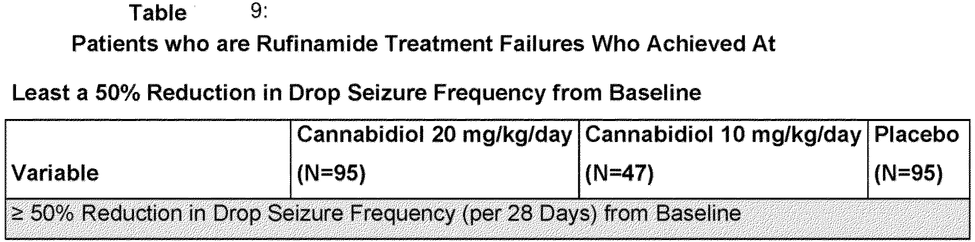

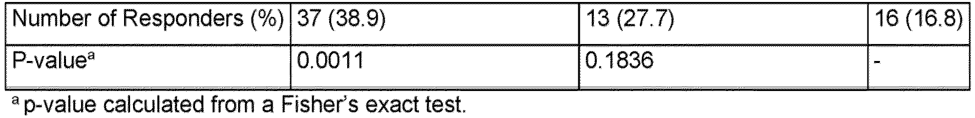

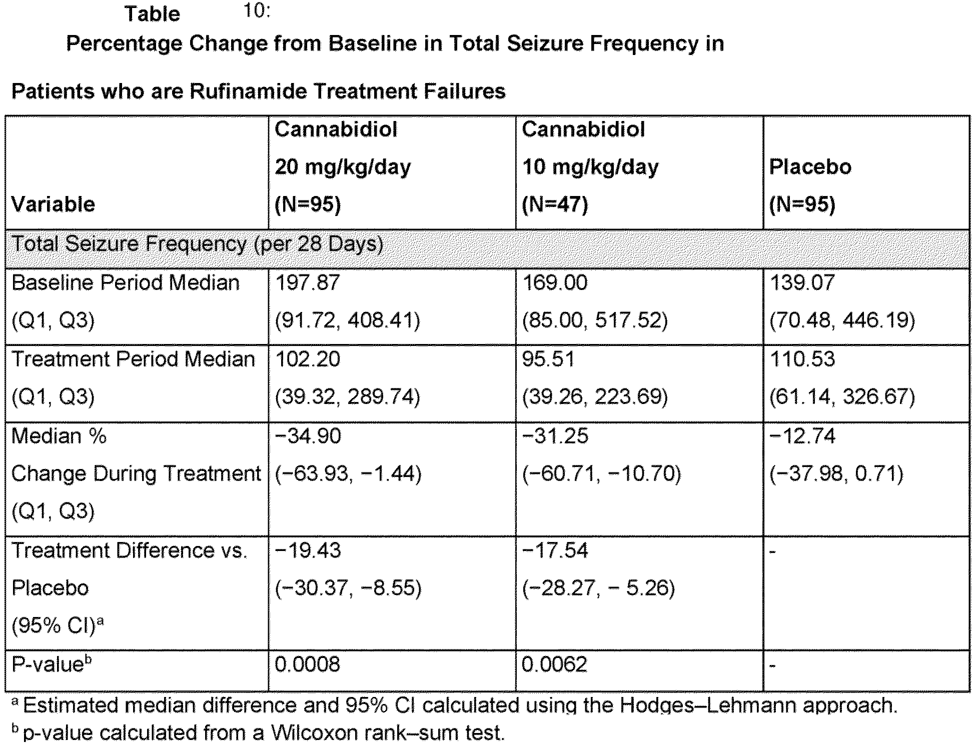

### Lamotrigine

Tables 11 to 13 below show the results in patients who have tried and failed lamotrigine, and those who are currently taking it but have uncontrolled seizures. There is a significantly greater reduction in both drop and total seizure frequency and a significantly greater proportion of patients with a ≥ 50% reduction in drop seizure frequency at both doses of cannabidiol compared with placebo.

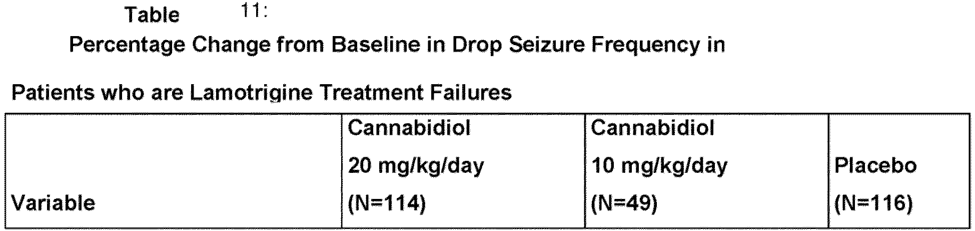

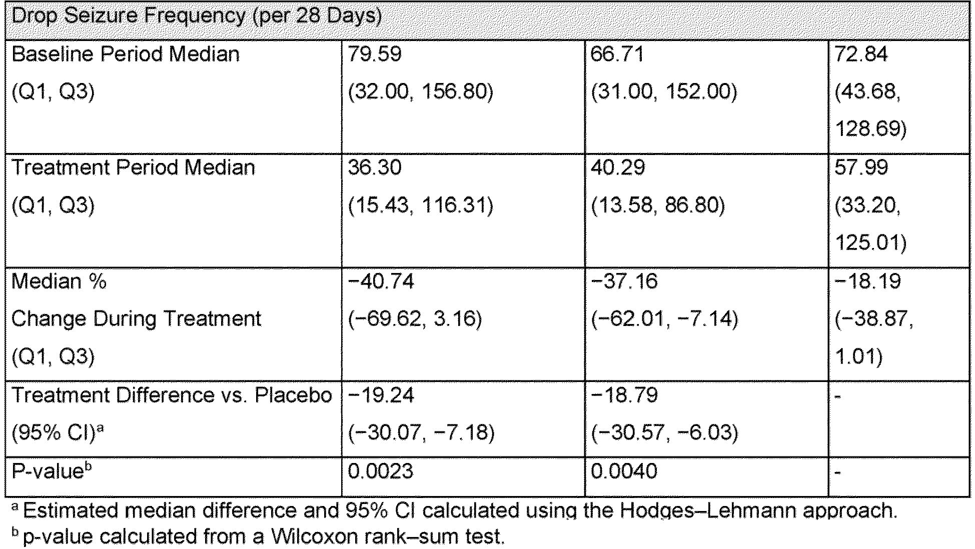

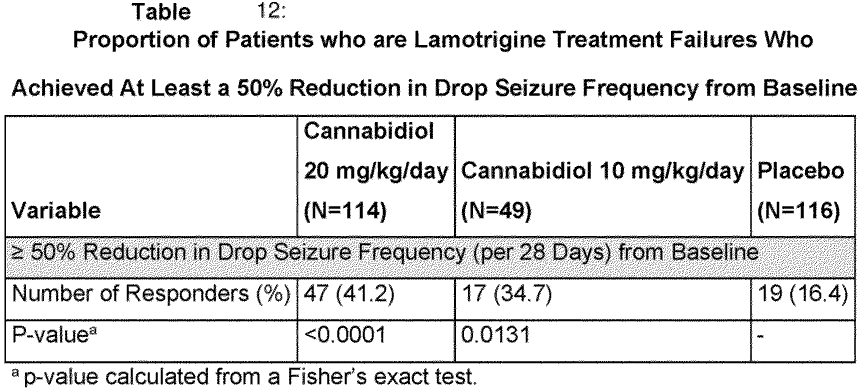

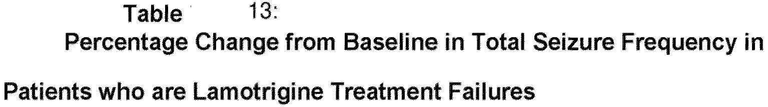

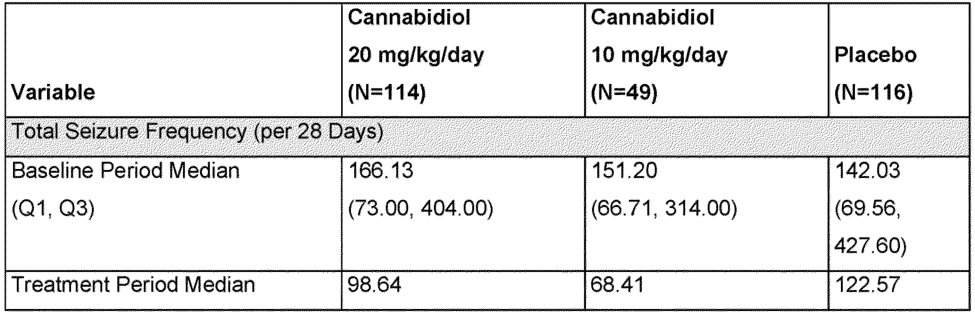

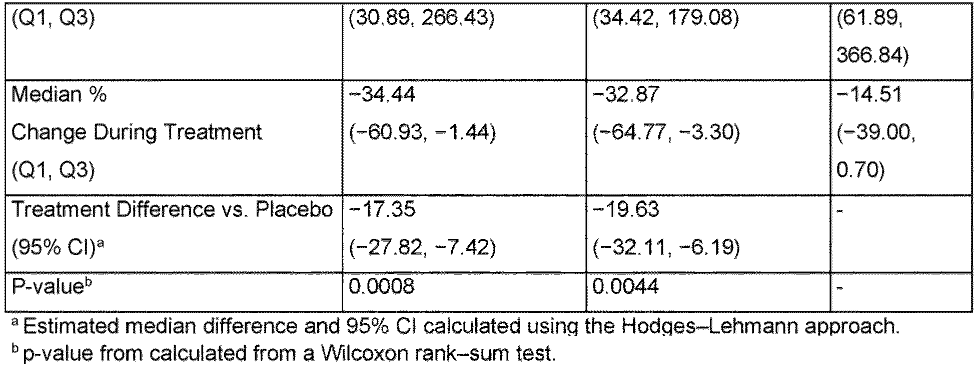

### Topiramate

Tables 14 to 16 below show the results in patients who have tried and failed topiramate, and those who are currently taking it but have uncontrolled seizures. There is a significantly greater reduction in both drop and total seizure frequency at both doses of cannabidiol compared with placebo, and a greater proportion of patients with a ≥ 50% reduction in drop seizure frequency at both doses of cannabidiol compared with placebo (reaching statistical significance for the 20 mg/kg/day dose but only marginal statistical significance for the 10 mg/kg/day dose).

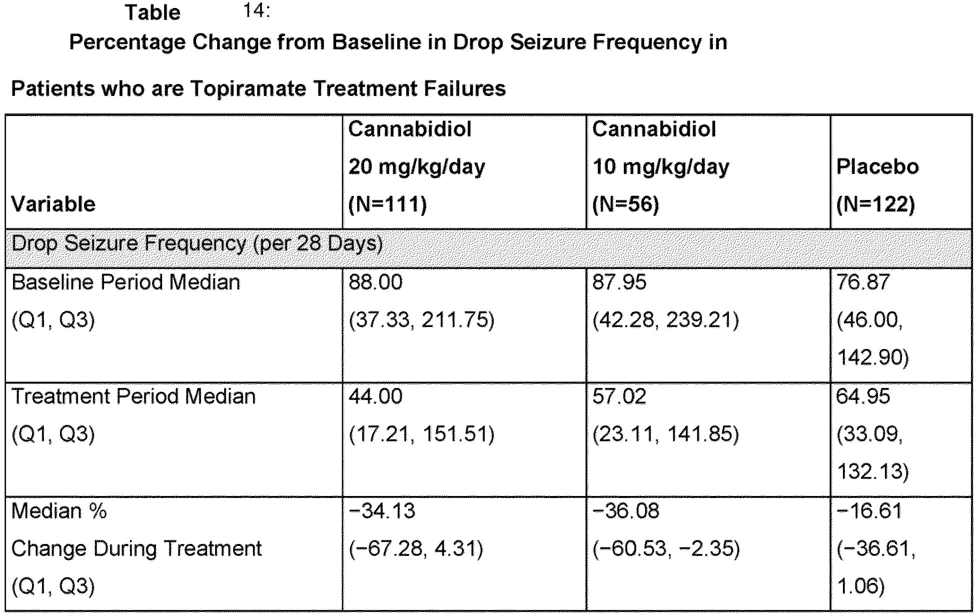

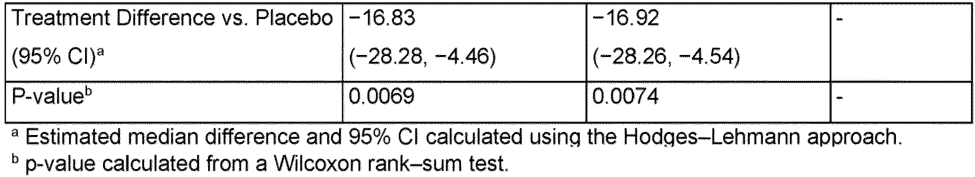

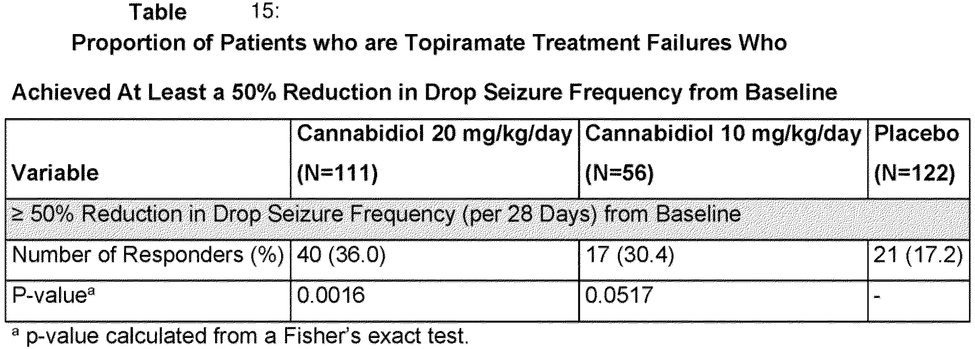

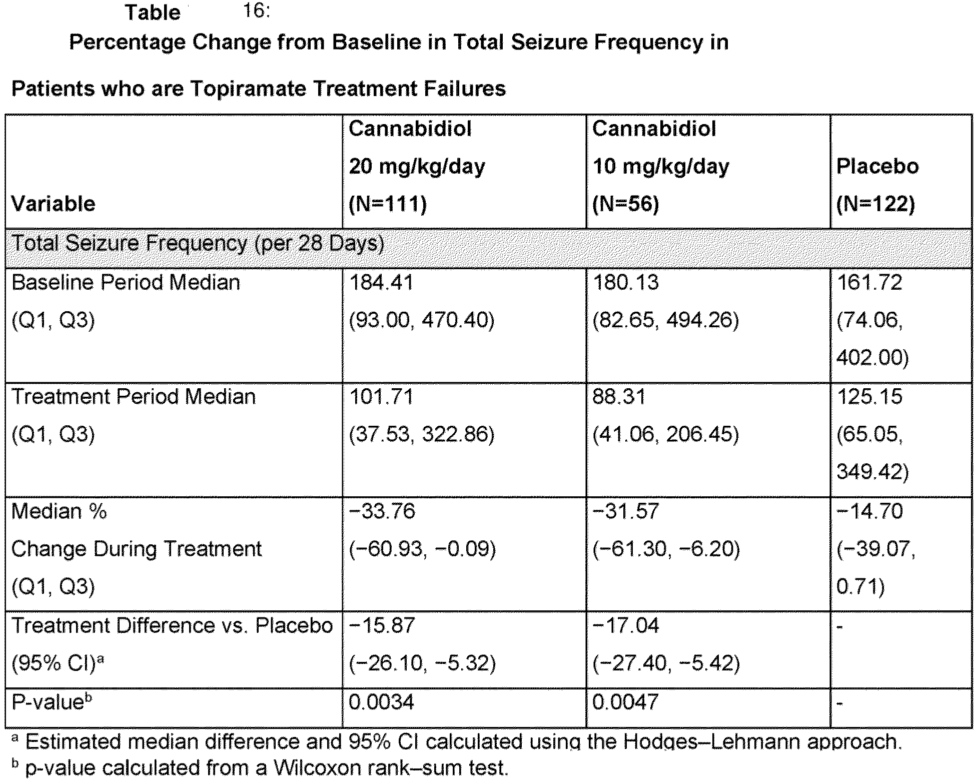

### Felbamate

Tables 17 to 19 below show the results in patients who have tried and failed felbamate, and those who are currently taking it but have uncontrolled seizures. There is a marginally statistically significantly greater reduction in drop seizure frequency and a statistically significantly greater proportion of patients with a ≥ 50% reduction in drop seizure frequency at the 20 mg/kg dose of cannabidiol compared with placebo.

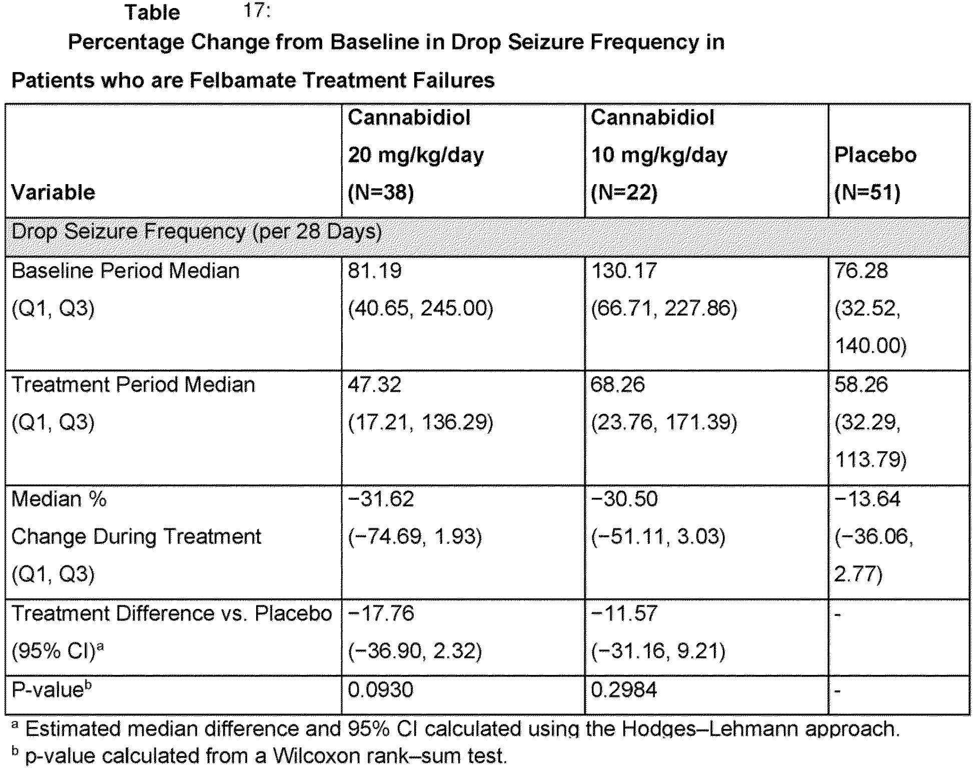

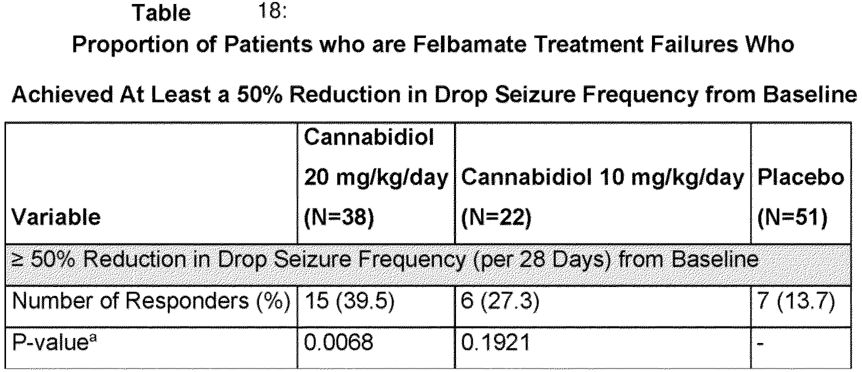

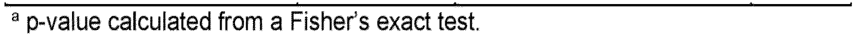

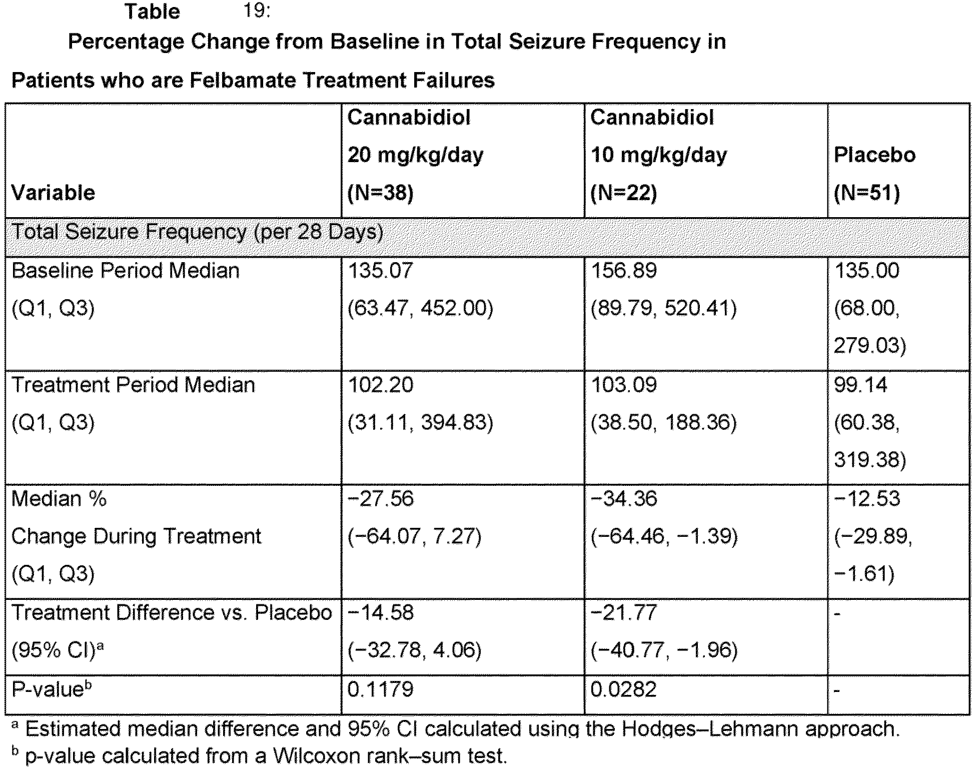

### Conclusions

Pooled data from two well-controlled, multi-centre, multi-national randomised studies show that patients who are taking without seizure control, or who have previously taken and have since stopped, the AEDs authorised for use in LGS in the EU, go on to achieve significant benefit from CBD. This effect is most significant at the daily dose of 20 mg/kg/day.

These data show that add-on treatment with CBD is able to provide a clinically relevant advantage over and above that obtained by lamotrigine, rufinamide, felbamate and topiramate. This clinically relevant advantage is consistent with the different mode of action of cannabidiol to those of these AEDs.

Surprisingly the combined use of CBD with one or more of the medications approved to treat LGS, namely rufinamide, lamotrigine, topiramate or felbamate enables a statistically significant reduction in drop and total seizure frequency in patients deemed to be treatment failures on their existing medication.

## Claims

1. Cannabidiol (CBD) for use in the treatment of drop seizures associated with Lennox-Gastaut syndrome (LGS) **characterised in that** the patient with LGS is deemed to be a treatment failure on lamotrigine; wherein the CBD is in the form of a highly purified extract of cannabis which comprises at least 98% (w/w) CBD or is present as a synthetic compound.

2. Cannabidiol (CBD) for use according to claim 1, wherein the highly purified extract comprises less than 0.15% THC.

3. Cannabidiol (CBD) for use according to claim 1, wherein the extract further comprises up to 1% CBDV.

4. Cannabidiol (CBD) for use according to any of the preceding claims, wherein the dose of CBD is below 50 mg/kg/day.

5. Cannabidiol (CBD) for use according to any of the preceding claims, wherein the dose of CBD is greater than 20 mg/kg/day.

## Patentansprüche

1. Cannabidiol (CBD) zur Verwendung bei der Behandlung von mit dem Lennox-Gestaut-Syndrom (LGS) verbundenen Sturzanfällen, **dadurch gekennzeichnet, dass** bei dem Patienten mit LGS die Behandlung mit Lamotrigin als erfolglos angesehen wird; wobei das CBD in Form eines hochgereinigten Cannabisextrakts vorliegt, das mindestens 98 (Gew.-)% CBD umfasst, oder als eine synthetische Verbindung vorhanden ist.

2. Cannabidiol (CBD) zur Verwendung nach Anspruch 1, wobei das hochgereinigte Extrakt weniger als 0,15% THC umfasst.

3. Cannabidiol (CBD) zur Verwendung nach Anspruch 1, wobei das Extrakt ferner bis zu 1% CBDV umfasst.

4. Cannabidiol (CBD) zur Verwendung nach einem der vorhergehenden Ansprüche, wobei die Dosis von CBD weniger als 50 mg/kg/Tag beträgt.

5. Cannabidiol (CBD) zur Verwendung nach einem der vorhergehenden Ansprüche, wobei die Dosis von CBD mehr als 20 mg/kg/Tag beträgt.

## Revendications

1. Cannabidiol (CBD) destiné à être utilisé dans le traitement des crises de chute associées au syndrome de Lennox-Gastaut (LGS) **caractérisé en ce que** le patient atteint du LGS est considéré comme étant un échec de traitement sur la lamotrigine ; ledit CBD se présentant sous la forme d'un extrait hautement purifié de cannabis qui comprend au moins 98 % (p/p) de CBD ou étant présent sous la forme d'un composé synthétique.

2. Cannabidiol (CBD) destiné à être utilisé selon la revendication 1, ledit extrait hautement purifié comprenant moins de 0,15 % de THC.

3. Cannabidiol (CBD) destiné à être utilisé selon la revendication 1, ledit extrait comprenant en outre jusqu'à 1 % de CBDV.

4. Cannabidiol (CBD) destiné à être utilisé selon l'une quelconque des revendications précédentes, ladite dose de CBD étant inférieure à 50 mg/kg/jour.

5. Cannabidiol (CBD) destiné à être utilisé selon l'une quelconque des revendications précédentes, ladite dose de CBD étant supérieure à 20 mg/kg/jour.
